(19) Europäisches Patentamt
European Patent Office
Office européen des brevets

(11) Veröffentlichungsnummer: **0 001 546**
**A1**

(12) **EUROPÄISCHE PATENTANMELDUNG**

(21) Anmeldenummer: 78100615.0

(22) Anmeldetag: 07.08.78

(51) Int. Cl.²: C 07 D 209/14
A 61 U 31/40

(30) Priorität: 03.09.77 DE 2739723

(43) Veröffentlichungstag der Anmeldung: 02.05.79 Patentblatt 79/9

(84) Benannte Vertragsstaaten: BE CH DE FR GB-NL SE

(71) Anmelder: Merck Patent Gesellschaft mit beschränkter Haftung
Frankfurter Strasse 250
D-6100 Darmstadt(DE)

(72) Erfinder: Jonas, Rochus, Dr.
Graupnerweg 26
D-6100 Darmstadt(DE)

(72) Erfinder: Schliep, Hans-Jochen, Dr.
Weingartenstrasse 16
D-6101 Traisa(DE)

(72) Erfinder: Schorscher, Ernst, Dr.
Im Fiedlersee 1
D-6100 Darmstadt-Arheilgen(DE)

(54) **2-Guanidinomethyl-indoline; Verfahren zu ihrer Herstellung und ihre pharmazeutische Verwendung.**

(57) 2-Guanidinomethyl-indoline der allgemeinen Formel I

I 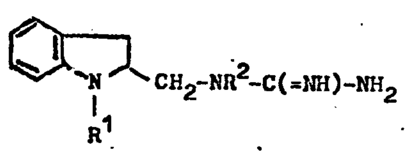

worin

$R^1$ und $R^2$ jeweils H oder Alkyl mit 1 - 6 C-Atomen bedeuten, sowie ihre physiologisch unbedenklichen Säureadditionssalze zeigen Wirkungen auf den Kreislauf, insbesondere blutdrucksenkende Wirkungen. Sie können z.B. hergestellt werden, indem man ein 2-Aminomethylindolin der allgemeinen Formel II

II 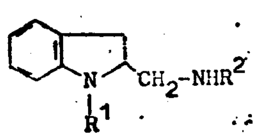

worin

$R^1$ und $R^2$ die bei Formel I angegebene Bedeutung haben oder ein Säureadditionssalz einer solchen Verbindung mit einer Verbindung der allgemeinen Formel III

$$NR^3{=}CR^4{-}NH_2$$

III

worin

$R^3$ H,

$R^4$ Alkyloxy, Alkylmercapto, 3,5-Dialkyl-1-pyrazolyl worin die Alkylgruppen jeweils 1 - 4 C-Atome haben oder NC-NH- oder

$R^3$ und $R^4$ zusammen eine C-N-Bindung bedeuten,

oder einem Säureadditionssalz einer solchen Verbindung umsetzt.

EP 0 001 546 A1

Croydon Printing Company Ltd

Merck Patent Gesellschaft
mit beschränkter Haftung
D a r m s t a d t

BEZEICHNUNG GEÄNDERT
siehe Titelseite

**2-Guanidinomethyl-indoline und Verfahren zu ihrer Herstellung**

Die Erfindung betrifft 2-Guanidinomethyl-indoline der allgemeinen Formel I

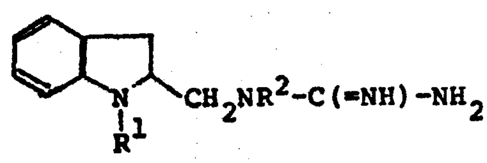

I

worin

$R^1$ und $R^2$ jeweils H oder Alkyl mit 1 - 6 C-Atomen bedeuten,

sowie deren physiologisch unbedenkliche Säureadditionssalze.

Der Erfindung lag die Aufgabe zugrunde, neue Verbindungen aufzufinden, die zur Herstellung von Arzneimitteln verwendet werden können. Diese Aufgabe wurde durch die Bereitstellung der Verbindungen der Formel I gelöst.

Es wurde gefunden, daß die Verbindungen der Formel I und ihre physiologisch unbedenklichen Säureadditionssalze bei guter Verträglichkeit wertvolle pharmakologische Eigenschaften besitzen. So zeigen sich beispielsweise Wirkungen auf den Kreislauf, insbesondere blutdrucksenkende Wirkungen; die Substanzen wirken z.B. am wachen nephrogen hypertonen Hund (Methodik vgl. z.B. Zeitschrift f. ges. exptl. Med., Band <u>130</u>, Seite 513 ff. (1959)) im Dauerversuch in Dosen ab 1 mg/kg per os dosisabhängig blutdrucksenkend. Bei der malignen Hypertonie des Hundes (Blutdruck systolisch 240 mm Hg oder höher, diastolisch 150 mm oder höher) wirken höhere Dosen lebensrettend für die Tiere.

Die Verbindungen der Formel I und ihre physiologisch unbedenklichen Säureadditionssalze können daher als Arzneimittel in der Human- und Veterinärmedizin und auch als Zwischenprodukte zur Herstellung anderer Arzneimittel verwendet werden.

Gegenstand der Erfindung sind die Verbindungen der Formel I und deren physiologisch unbedenkliche Säureadditionssalze.

In dieser Formel bedeuten die Reste $R^1$ und $R^2$, die gleich oder verschieden sein können, vorzugsweise H oder Alkyl mit 1 - 3 C-Atomen, insbesondere H, Methyl oder Äthyl, ferner n-Propyl oder Isopropyl, außerdem Butyl wie n-Butyl, Isobutyl, sek.-Butyl oder tert.-Butyl, Pentyl wie n-Pentyl, Isopentyl, Neopentyl, Hexyl wie n-Hexyl oder Isohexyl.

Gegenstand der Erfindung ist ferner ein Verfahren zur Herstellung der Verbindungen der Formel I, dadurch gekennzeichnet, daß man ein 2-Aminomethylindolin der allgemeinen Formel II

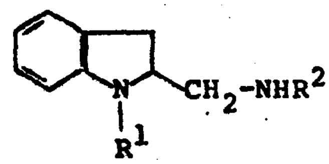

II

worin

$R^1$ und $R^2$ die bei Formel I angegebene Bedeutung haben

oder ein Säureadditionssalz einer solchen Verbindung mit einer Verbindung der allgemeinen Formel III

$$NR^3{=}CR^4{-}NH_2 \qquad III$$

worin

$R^3$ H,

$R^4$ Alkyloxy, Alkylmercapto, 3,5-Dialkyl-1-pyrazolyl (worin die Alkylgruppen jeweils 1 - 4 C-Atome haben) oder NC-NH- oder

$R^3$ und $R^4$ zusammen eine C-N-Bindung bedeuten,

oder einem Säureadditionssalz einer solchen Verbindung umsetzt oder daß man eine Verbindung, die der allgemeinen Formel I entspricht, jedoch an Stelle eines oder mehrerer H-Atome eine oder mehrere reduktiv abspaltbare Gruppe(n) und/oder Doppelbindung(en) enthält, insbesondere eine von I verschiedene Verbindung der allgemeinen Formel IV

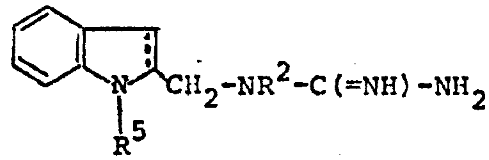 IV

worin sich in der durch eine punktierte Linie bezeichneten Stellung eine zusätzliche C-C-Bindung befinden kann,

$R^5$ einen hydrogenolytisch abspaltbaren Rest, insbesondere Benzyl, oder $R^1$ bedeutet und

$R^1$ und $R^2$ die bei Formel I angegebene Bedeutung haben,

oder ein Säureadditionssalz einer solchen Verbindung mit einem Reduktionsmittel behandelt und daß man gegebenenfalls die erhaltene Verbindung durch Behandeln mit einer Säure in ein physiologisch unbedenkliches Säureadditionssalz überführt.

Die Herstellung der Verbindungen der Formel I erfolgt im übrigen nach an sich bekannten Methoden, wie sie in der Literatur (z.B. in den Standardwerken wie Houben-Weyl, Methoden der Organischen Chemie, Georg-Thieme-Verlag, Stuttgart; Organic Reactions, John Wiley & Sons, Inc., New York) beschrieben sind, und zwar unter den für die genannten Umsetzungen bekannten und geeigneten Reaktionsbedingungen. Dabei kann man auch von an sich bekannten, hier nicht näher erwähnten Varianten Gebrauch machen.

Die Verbindungen der Formel I werden vorzugsweise durch Umsetzung der Verbindungen der Formeln II und III erhältlich.

Die 2-Aminomethylindoline der Formel II sind neu; sie können jedoch nach an sich bekannten Methoden hergestellt werden. Beispielsweise kann man Indolin-2-carbonsäureester mit $LiAlH_4$ zu 2-Hydroxymethylindolinen reduzieren und diese mit $SOCl_2$ oder $PBr_3$ zu 2-Chlormethyl- oder 2-Brommethylindolinen umsetzen; Reaktion mit Phthalimidkalium und anschließende Solvolyse führt zu den 2-Aminomethylindolinen

der Formel II ($R^2$ = H). Die 2-Aminomethylindoline der Formel II ($R^2$ = Alkyl mit 1 - 6 C-Atomen) sind daraus durch Alkylierung oder durch Acylierung (z.B. Acetylierung) und nachfolgende Reduktion erhältlich.

Die Verbindungen der Formel III sind in der Regel bekannt und umfassen insbesondere Cyanamid, Dicyandiamid, O-Alkylisoharnstoffe, S-Alkylisothioharnstoffe oder 3,5-Dialkylpyrazol-1-carboxamidine, worin die Alkylgruppen 1 - 4 C-Atome haben, vorzugsweise aber Methyl bedeuten.

Bevorzugt sind die S-Alkylisothioharnstoffe und, insbesondere zur Herstellung der Verbindungen der Formel I, worin $R^2$ Alkyl mit 1 - 6 C-Atomen bedeutet, Cyanamid.

Als Ausgangsstoffe der Formel IV eignen sich insbesondere die Indolderivate, in denen die punktierte Linie eine zusätzliche C-C-Bindung und $R^5$ = $R^1$ bedeutet, sowie die Indolinderivate, in denen $R^5$ einen hydrogenolytisch entfernbaren Rest bedeutet, z.B. Benzyl, substituierte Benzylgruppen wie Tolylmethyl, Diphenylmethyl, Carbobenzoxy. Die Verbindungen der Formel IV sind neu; sie sind analog den Verbindungen der Formel II erhältlich, wenn man von 1-$R^5$-Indol- oder 1-$R^5$-Indolin-2-carbonsäureestern ausgeht.

Als Säureadditionssalze der Verbindungen II, III und IV eignen sich z.B. deren Hydrochloride, Sulfate oder Nitrate.

Die Verbindung I wird z.B. hergestellt, indem man das Amin II als Base oder in Form des Sulfats oder eines anderen Salzes mit Cyanamid in der Schmelze bei Temperaturen zwischen 100° und 200°, vorzugsweise zwischen 110° und 150°, oder in einem inerten Lösungsmittel, z.B. Kohlenwasserstoffen wie Benzol, Toluol, Alkoholen wie Methanol, Äthanol, Propanol, Butanol, hochsiedenden Äthern wie Äthylenglykolmono- oder dialkyläthern, Wasser

oder Alkohol/Wasser-Gemischen, bei Temperaturen zwischen etwa 20° und der Siedetemperatur des Lösungsmittels, besonders zwischen etwa 110° und etwa 150° umsetzt. Anstelle von Cyanamid kann bei der Reaktion auch Dicyandiamid unter sonst gleichen Bedingungen verwendet werden; dabei entsteht Cyanamid in situ.

Die Verbindung I kann ferner erhalten werden durch Umsetzung des Amins II mit einem Säureaddditionssalz eines S-Alkyl-isothioharnstoffs oder eines O-Alkyl-isoharnstoffs, vorzugsweise in Gegenwart eines inerten Lösungsmittels wie Wasser, Aceton, Dioxan, Alkoholen sowie anderen mit Wasser mischbaren Lösungsmitteln, wobei das Einhalten einer bestimmten Temperatur nicht erforderlich ist und die Reaktion z.B. zwischen 0° und dem Siedepunkt des verwendeten Lösungsmittels ablaufen kann. Weiterhin kann die Verbindung II mit einem Salz eines 3,5-Dialkylpyrazol-1-carboxamidins, z. B. 3,5-Dimethylpyrazol-1-carboxamidinnitrat, vorzugsweise in einem inerten Lösungsmittel wie Wasser, Alkoholen oder anderen mit Wasser mischbaren Lösungsmitteln oder auch ohne Lösungsmittel bei Temperaturen zwischen 50° und 150° zur Verbindung I umgesetzt werden.

Außerdem läßt sich auch eine Verbindung der Formel IV oder ein Säureadditionssalz einer solchen Verbindung auf chemischem oder katalytischem Wege zur Verbindung I (bzw. zu einem Säureadditionssalz von I) reduzieren.

Die chemische Reduktion läßt sich vorzugsweise durchführen mit Natriumborhydrid in Essigsäure oder mit Diboran in Kohlenwasserstoffen, Äthern, cyclischen Äthern, insbesondere Dioxan, Tetrahydrofuran oder Essigsäure, bei Temperaturen zwischen -70° und +100°, vorzugsweise zwischen -40° und +40°.

Die katalytische Reduktion erfolgt z.B. in Gegenwart von Katalysatoren wie Platin, Nickel, Kupfer, Palladium oder deren Salzen in Lösungsmitteln wie Alkoholen, Dioxan, Äthylacetat oder Essigsäure, vorzugsweise in Essigsäure/Salzsäure mit Palladium/Bariumsulfat. N-Benzylgruppen werden hydrogenolytisch vorzugsweise an Palladium/Kohle abgespalten. Die Temperaturen liegen dabei vorzugsweise zwischen etwa $0^o$ und $100^o$, insbesondere zwischen etwa 40 und etwa $70^o$, wobei sowohl bei Normaldruck als auch unter erhöhtem Druck (bis zu etwa 200 at) gearbeitet werden kann.

Eine nach der Erfindung erhaltene freie Base der Formel I kann mit einer Säure in das zugehörige Säureadditionssalz übergeführt werden. Je nach der zugefügten Säuremenge kann man die Mono- oder Di-säure-additionssalze, z.B. Mono- oder Dihydrochloride, Hemisulfate oder Sulfate, erhalten. Für diese Umsetzung eignen sich Säuren, die physiologisch unbedenkliche Salze liefern. So eignen sich anorganische oder organische Säuren, z.B. aliphatische, alicyclische, aromatische, araliphatische oder heterocyclische ein- oder mehrbasige Carbonsäuren oder Sulfonsäuren, im einzelnen z.B. Salzsäure, Bromwasserstoffsäure, Schwefelsäure, Phosphorsäuren wie Orthophosphorsäure, Salpetersäure, Ameisensäure, Essigsäure, Propionsäure, Pivalinsäure, Diäthylessigsäure, Malonsäure, Bernsteinsäure, Pimelinsäure, Fumarsäure, Maleinsäure, Milchsäure, Weinsäure, Äpfelsäure, Benzoesäure, Salicylsäure, 2-Phenylpropionsäure, Citronensäure, Gluconsäure, Ascorbinsäure, Nicotinsäure, Isonicotinsäure, Methan- oder Äthansulfonsäure, Äthandisulfonsäure, 2-Hydroxyäthansulfonsäure, Benzolsulfonsäure, p-Toluolsulfonsäure, Naphthalin-mono- und -disulfonsäuren.

Die freien Basen der Formel I können, falls gewünscht, aus ihren Salzen durch Behandlung mit starken Basen, wie Natrium- oder Kaliumhydroxid, Natrium- oder Kaliumcarbonat, in Freiheit gesetzt werden.

Die Verbindungen der Formel I können ein oder mehrere Asymmetriezentren besitzen. Sie können daher bei ihrer Herstellung als Racemate oder, falls optisch aktive Ausgangsstoffe verwendet werden, auch in optisch aktiver Form erhalten werden.

Weisen die Verbindungen zwei oder mehr Asymmetriezentren auf, dann fallen sie bei der Synthese im allgemeinen als Gemische von Racematen an, aus denen man die einzelnen Racemate, beispielsweise durch Umkristallisieren aus inerten Lösungsmitteln, in reiner Form isolieren kann.

Erhaltene Racemate können, falls erwünscht, nach an sich bekannten Methoden mechanisch oder chemisch in ihre optischen Antipoden getrennt werden. Vorzugsweise werden aus dem Racemat durch Umsetzung mit einem optisch aktiven Trennmittel Diastereomere gebildet. Als Trennmittel eignen sich z.B. optisch aktive Säuren, wie Weinsäure, Dibenzoylweinsäure, Diacetylweinsäure, Camphersulfonsäuren, Mandelsäure, Äpfelsäure oder Milchsäure.

Die neuen Verbindungen der Formel I und ihre physiologisch unbedenklichen Säureadditionssalze können zur Herstellung pharmazeutischer Präparate verwendet werden, indem man sie zusammen mit mindestens einem Träger- oder Hilfsstoff und gegebenenfalls zusammen mit einem oder mehreren weiteren Wirkstoff(en) in eine geeignete Dosierungsform bringt. Die so erhaltenen Zubereitungen können als Arzneimittel in der Human- oder Veterinärmedizin eingesetzt werden. Als Trägersubstanzen kommen organische oder anorganische Stoffe in Frage, die sich für die enterale,

parenterale oder topikale Applikation eignen und mit den neuen Verbindungen nicht reagieren, beispielsweise Wasser, pflanzliche Öle, Benzylalkohole, Polyäthylenglykole, Gelatine, Kohlehydrate wie Lactose oder Stärke, Magnesiumstearat, Talk, Vaseline. Zur enteralen Applikation dienen insbesondere Tabletten, Dragees, Kapseln, Sirupe, Säfte, Tropfen oder Suppositorien, zur parenteralen Applikation Lösungen, vorzugsweise ölige oder wässerige Lösungen, ferner Suspensionen, Emulsionen oder Implantate, für die topikale Anwendung Salben, Cremes oder Puder. Die neuen Verbindungen können auch lyophilisiert und die erhaltenen Lyophilisate z.B. zur Herstellung von Injektionspräparaten verwendet werden. Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks- und/oder Aromastoffe enthalten. Sie können, falls erwünscht, auch einen oder mehrere weitere Wirkstoffe enthalten.

Die erfindungsgemäßen Substanzen werden in der Regel in Analogie zu bekannten, im Handel befindlichen Antihypertonika (z.B. Guanethidin) verabreicht, vorzugsweise in Dosierungen zwischen etwa 1 und 100 mg, insbesondere zwischen 5 und 50 mg pro Dosierungseinheit. Die tägliche Dosierung liegt vorzugsweise zwischen etwa 0,02 und 5 mg/kg Körpergewicht. Die spezielle Dosis für jeden bestimmten Patienten hängt jedoch von den verschiedensten Faktoren, ab, beispielsweise von der Wirksamkeit der eingesetzten speziellen Verbindung, vom Alter, Körpergewicht, allgemeinem Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

Jede der in den folgenden Beispielen genannten Verbindungen der Formel I ist zur Herstellung von pharmazeutischen Zubereitungen besonders geeignet.

Beispiel 1

Eine Lösung von 17,6 g 1-Äthyl-2-aminomethyl-indolin (erhältlich durch N-Äthylierung von Indolin-2-carbonsäureäthylester zu 1-Äthyl-indolin-2-carbonsäureäthylester, $LiAlH_4$-Reduktion zu 1-Äthyl-2-hydroxymethyl-indolin, Reaktion mit $SOCl_2$ zu 1-Äthyl-2-chlormethyl-indolin, Umsetzung mit Phthalimid-kalium zur 1-Äthyl-2-phthalimidomethyl-indolin und Hydrolyse) in 60 ml Äthanol wird mit einer Lösung von 22,4 g S-Methyl-isothioharnstoff-hemisulfat in 50 ml Wasser versetzt und zwei Tage bei 20° gerührt. Man kühlt ab, filtriert, wäscht mit Aceton und erhält 1-Äthyl-2-guanidinomethyl-indolin in Form des Hemisulfats, F. 199°. Mit Natronlauge ist daraus die freie Base (Öl) erhältlich.

Beispiele 2 bis 10

Analog Beispiel 1 erhält man aus 2-Aminomethylindolin, 1-Methyl-, 1-n-Propyl-, 1-Isopropyl-, 1-n-Butyl-, 1-Isobutyl-, 1-n-Pentyl-, 1-Isopentyl- oder 1-n-Hexyl-2-aminomethylindolin:

2. 2-Guanidinomethyl-indolin.
3. 1-Methyl-2-guanidinomethyl-indolin, Hemisulfat, F. 200°.
4. 1-n-Propyl-2-guanidinomethyl-indolin.
5. 1-Isopropyl-2-guanidinomethyl-indolin.
6. 1-n-Butyl-2-guanidinomethyl-indolin.
7. 1-Isobutyl-2-guanidinomethyl-indolin.
8. 1-n-Pentyl-2-guanidinomethyl-indolin.
9. 1-Isopentyl-2-guanidinomethyl-indolin.
10. 1-n-Hexyl-2-guanidinomethyl-indolin.

Beispiel 11

Man versetzt eine Lösung von 20,4 g 1-Äthyl-2-äthylaminomethyl-indolin (erhältlich aus 1-Äthyl-2-chlormethylindolin und Äthylamin) in 40 ml Methanol mit Schwefelsäure bis zum pH 8, gibt eine Lösung von 5,4 g Cyanamid in 8 ml Wasser hinzu und stellt mit wässerigem Ammoniak erneut auf pH 8 ein. Nach 12-stündigem Kochen wird unter Eiskühlung Schwefelsäure bis pH 6 zugegeben, eingedampft und der Rückstand mit Aceton zur Kristallisation gebracht. Man erhält 1-Äthyl-2-(1-äthyl-guanidinomethyl)-indolin-hemisulfat, F. 230°.

Beispiele 12 bis 51

Analog Beispiel 11 erhält man aus den entsprechenden 2-Alkylaminomethyl-indolinen mit Cyanamid:

12. 2-(1-Methyl-guanidinomethyl)-indolin.
13. 2-(1-Äthyl-guanidinomethyl)-indolin.
14. 2-(1-n-Propyl-guanidinomethyl)-indolin.
15. 2-(1-Isopropyl-guanidinomethyl)-indolin.
16. 2-(1-n-Butyl-guanidinomethyl)-indolin.
17. 2-(1-Isobutyl-guanidinomethyl)-indolin.
18. 2-(1-n-Pentyl-guanidinomethyl)-indolin.
19. 2-(1-Isopentyl-guanidinomethyl)-indolin.
20. 2-(1-n-Hexyl-guanidinomethyl)-indolin.
21. 1-Methyl-2-(1-methyl-guanidinomethyl)-indolin.
22. 1-Methyl-2-(1-äthyl-guanidinomethyl)-indolin.
23. 1-Methyl-2-(1-n-propyl-guanidinomethyl)-indolin.
24. 1-Methyl-2-(1-isopropyl-guanidinomethyl)-indolin.
25. 1-Methyl-2-(1-n-butyl-guanidinomethyl)-indolin.
26. 1-Methyl-2-(1-isobutyl-guanidinomethyl)-indolin.

27. 1-Methyl-2-(1-n-pentyl-guanidinomethyl)-indolin.
28. 1-Methyl-2-(1-isopentyl-guanidinomethyl)-indolin.
29. 1-Methyl-2-(1-n-hexyl-guanidinomethyl)-indolin.
30. 1-Äthyl-2-(1-methyl-guanidinomethyl)-isoindolin.
31. 1-Äthyl-2-(1-n-propyl-guanidinomethyl)-isoindolin.
32. 1-Äthyl-2-(1-isopropyl-guanidinomethyl)-isoindolin.
33. 1-Äthyl-2-(1-n-butyl-guanidinomethyl)-isoindolin.
34. 1-Äthyl-2-(1-isobutyl-guanidinomethyl)-isoindolin.
35. 1-Äthyl-2-(1-n-pentyl-guanidinomethyl)-isoindolin.
36. 1-Äthyl-2-(1-isopentyl-guanidinomethyl)-isoindolin.
37. 1-Äthyl-2-(1-n-hexyl-guanidinomethyl)-isoindolin.
38. 1-n-Propyl-2-(1-methyl-guanidinomethyl)-isoindolin.
39. 1-n-Propyl-2-(1-äthyl-guanidinomethyl)-isoindolin.
40. 1-Isopropyl-2-(1-methyl-guanidinomethyl)-isoindolin.
41. 1-Isopropyl-2-(1-äthyl-guanidinomethyl)-isoindolin.
42. 1-n-Butyl-2-(1-methyl-guanidinomethyl)-isoindolin.
43. 1-n-Butyl-2-(1-äthyl-guanidinomethyl)-isoindolin.
44. 1-Isobutyl-2-(1-methyl-guanidinomethyl)-isoindolin.
45. 1-Isobutyl-2-(1-äthyl-guanidinomethyl)-isoindolin.
46. 1-n-Pentyl-2-(1-methyl-guanidinomethyl)-isoindolin.
47. 1-n-Pentyl-2-(1-äthyl-guanidinomethyl)-isoindolin.
48. 1-Isopentyl-2-(1-methyl-guanidinomethyl)-isoindolin.
49. 1-Isopentyl-2-(1-äthyl-guanidinomethyl)-isoindolin.
50. 1-n-Hexyl-2-(1-methyl-guanidinomethyl)-isoindolin.
51. 1-n-Hexyl-2-(1-äthyl-guanidinomethyl)-isoindolin.

**Beispiel 52**

Ein Gemisch aus 25,3 g 1-Äthyl-2-äthylaminomethyl-indolin-hemisulfat und 25 g Cyanamid wird 20 Minuten auf 120$^{o}$ erwärmt. Nach dem Abkühlen versetzt man mit Aceton und saugt ab. Die Kristalle werden in eiskalter Natriumbicarbonatlösung aufgenommen und die wässerige Phase mit Chloroform extrahiert. Die nach dem Trocknen und Eindampfen der Chloroformphase erhaltene ölige Base wird in wenig Methanol gelöst und die Lösung mit

Schwefelsäure auf pH 6 eingestellt. Man dampft ein, reibt den Rückstand mit Aceton an und erhält 1-Äthyl-2-(1-äthyl-guanidinomethyl)-indolin-hemisulfat, F. 230°.

Beispiel 53

Eine Lösung von 26,5 g 1-Äthyl-2-guanidinomethyl-indol-hemisulfat (erhältlich aus Indol-2-carbonsäure-äthylester über 1-Äthyl-indol-2-carbonsäureäthylester, 1-Äthyl-2-hydroxymethyl-indol, 1-Äthyl-2-chlormethyl-indol, 1-Äthyl-2-phthalimidomethyl-indol und 1-Äthyl-2-aminomethyl-indol) in einem Gemisch von 300 ml Essigsäure und 70 ml 2 n Schwefelsäure wird bei 60° mit 10 g Palladium/Bariumsulfat (5 %) unter Normaldruck hydriert. Nach dem Ende der Wasserstoffaufnahme wird die Lösung filtriert. Man dampft ein und erhält 1-Äthyl-2-guanidinomethyl-indolin-hemisulfat, F. 199°.

Beispiel 54

Eine Lösung von 26,5 g 1-Äthyl-2-guanidinomethyl-indol-hemisulfat in 100 ml Essigsäure wird bei 20° unter Rühren mit 23 g Natriumborhydrid versetzt. Man erwärmt kurz auf 60°, zersetzt mit Wasser, dampft ein, löst den Rückstand in Wasser, stellt auf pH 7 und extrahiert mit Äther. Nach dem Trocknen und Eindampfen des Extrakts wird mit verdünnter Schwefelsäure auf pH 6 gestellt, erneut, eingedampft und der Rückstand mit Aceton zur Kristallisation gebracht. Man erhält 1-Äthyl-2-guanidinomethyl-indolin-hemisulfat, F. 199°.

Beispiel 55

Man löst 10 g 1-Benzyl-2-guanidinomethyl-indolin-hemisulfat (erhältlich aus Indolin-2-carbonsäureäthylester durch N-Benzylierung, Reduktion und Reaktion mit $SOCl_2$ zu 1-Benzyl-2-chlormethyl-indolin, Umsetzung mit Phthalimid-kalium und Hydrolyse und Reaktion mit S-Methyl-isothioharnstoffhemisulfat) in 200 ml Äthanol und hydriert an 3 g 5 %igem Pd/C bei 20° und 1 at bis zum Stillstand. Nach dem Filtrieren und Eindampfen erhält man 2-Guanidinomethyl-indolin-hemisulfat.

Die nachstehenden Beispiele betreffen pharmazeutische Zubereitungen, die Guanidine der Formel I oder ihre Säureadditionssalze enthalten:

Beispiel A: Tabletten

Ein Gemisch von 1 kg 1-Äthyl-2-guanidinomethyl-indolin-sulfat, 4 kg Lactose, 1,2 kg Kartoffelstärke, 0,2 kg Talk und 0,1 kg Magnesiumstearat wird in üblicher Weise zu Tabletten gepreßt, derart, daß jede Tablette 10 mg Wirkstoff enthält.

Beispiel B: Dragees

Analog Beispiel A werden Tabletten gepreßt, die anschliessend in üblicher Weise mit einem Überzug aus Saccharose, Kartoffelstärke, Talk, Tragant und Farbstoff überzogen werden.

Beispiel C: Kapseln

5 kg 1-Methyl-2-guanidinomethyl-indolin-sulfat werden in üblicher WEise in Hartgelatinekapseln gefüllt, so daß jede Kapsel 20 mg des Wirkstoffs enthält.

Analog sind Tabletten, Dragees und Kapseln erhältlich, die einen oder mehrere der übrigen Wirkstoffe der Formel I und/oder ihre physiologisch unbedenklichen Säureadditionssalze enthalten.

Merck Patent Gesellschaft
mit beschränkter Haftung
D a r m s t a d t

## Patentansprüche:

1. 2-Guanidinomethyl-indoline der allgemeinen Formel I

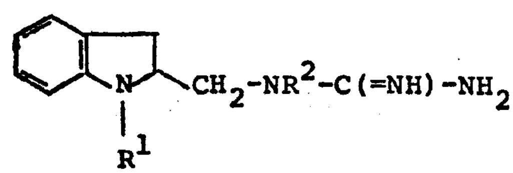

I

worin

$R^1$ und $R^2$ jeweils H oder Alkyl mit 1 - 6 C-Atomen bedeuten

und deren physiologisch unbedenkliche Säureadditionssalze.

2. a) 2-Guanidinomethyl-indolin und dessen physiologisch unbedenkliche Säureadditonssalze.
   b) 1-Methyl-2-guanidinomethyl-indolin und dessen physiologisch unbedenkliche Säureadditionssalze.
   c) 1-Äthyl-2-guanidinomethyl-indolin und dessen physiologisch unbedenkliche Säureadditionssalze.

d) 1-Äthyl-2-(1-äthyl-guanidinomethyl)-indolin und dessen physiologisch unbedenkliche Säureadditionssalze.

3. Verfahren zur Herstellung von 2-Guanidinomethylindolinen der allgemeinen Formel I

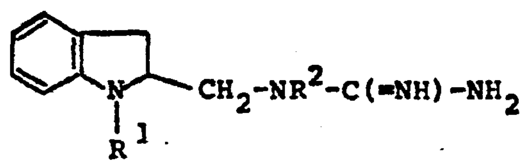

I

worin

$R^1$ und $R^2$ H oder Alkyl mit 1 - 6 C-Atomen bedeuten

und deren physiologisch unbedenklichen Säureadditionssalzen, dadurch gekennzeichnet, daß man ein 2-Aminomethylindolin der allgemeinen Formel II

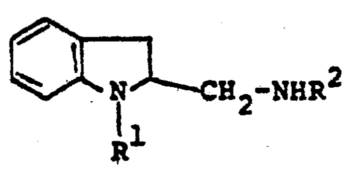

II

worin

$R^1$ und $R^2$ die bei Formel I angegebene Bedeutung haben

oder ein Säureadditionssalz einer solchen Verbindung mit einer Verbindung der allgemeinen Formel III

BAD ORIGINAL

$$NR^3{=}CR^4{-}NH_2 \qquad III$$

worin

$R^3$ H,

$R^4$ Alkyloxy, Alkylmercapto, 3,5-Dialkyl-1-pyrazolyl worin die Alkylgruppen jeweils 1 - 4 C-Atome haben oder NC-NH- oder

$R^3$ und $R^4$ zusammen eine C-N-Bindung bedeuten,

oder einem Säureadditionssalz einer solchen Verbindung umsetzt oder daß man eine Verbindung, die der allgemeinen Formel I entspricht, jedoch an Stelle eines oder mehrerer H-Atome eine oder mehrere reduktiv abspaltbare Gruppe(n) und/oder Doppelbindung(en) enthält, insbesondere eine von I verschiedene Verbindung der allgemeinen Formel IV

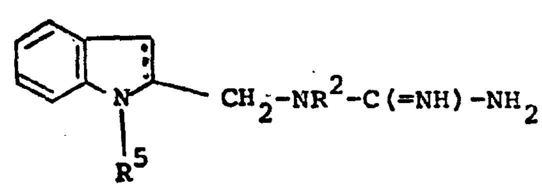

IV

worin sich in der durch eine punktierte Linie bezeichneten Stellung eine zusätzliche C-C-Bindung befinden kann,

$R^5$ einen hydrogenolytisch abspaltbaren Rest, insbesondere Benzyl, oder $R^1$ bedeutet und

$R^1$ und $R^2$ die bei Formel I angegebene Bedeutung haben

oder ein Säureadditionssalz einer solchen Verbindung mit einem Reduktionsmittel behandelt und daß man gegebenenfalls die erhaltene Verbindung durch Behandeln mit einer Säure in ein physiologisch unbedenkliches Säureadditionssalz überführt.

4. Verfahren zur Herstellung pharmazeutischer Präparate, dadurch gekennzeichnet, daß man eine Verbinding der Formel I und/oder eines ihrer physiologisch unbedenklichen Säureadditionssalze zusammen mit mindestens einem festen, flüssigen oder halbflüssigen Träger- oder Hilfsstoff und gegebenenfalls zusammen mit einem weiteren Wirkstoff in eine geeignete Dosierungsform bringt.

5. Pharmazeutische Zubereitung, enthaltend eine Verbindung der allgemeinen Formel I und/oder eines ihrer physiologisch unbedenklichen Säureadditionssalze.

Kurzreferat / Abstract

0001546


Neue 2-Guanidinomethyl-indoline der allgemeinen Formel I

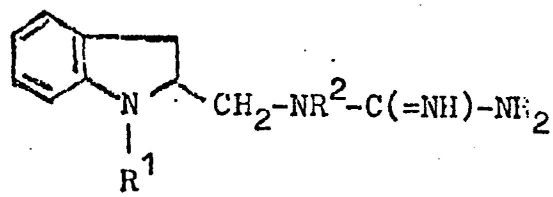

I

BAD ORIGINAL

worin
$R^1$ und $R^2$ jeweils H oder Alkyl mit 1 - 6 C-Atomen bedeuten,

sowie ihre physiologisch unbedenklichen Säureadditionssalze zeigen Wirkungen auf den Kreislauf, insbesondere blutdrucksenkende Wirkungen. Sie können z.B. hergestellt werden, indem man ein 2-Aminomethylindolin der allgemeinen Formel II

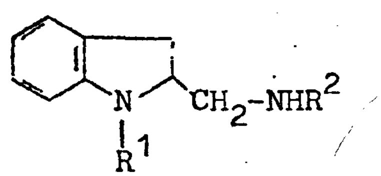

II

worin
$R^1$ und $R^2$ die bei Formel I angegebene Bedeutung haben

oder ein Säureadditionssalz einer solchen Verbindung mit einer Verbindung der allgemeinen Formel III

$NR^3{=}CR^4{-}NH_2$

III

worin
$R^3$ H,
$R^4$ Alkyloxy, Alkylmercapto, 3,5-Dialkyl-1-pyrazolyl worin die Alkylgruppen jeweils 1 - 4 C-Atome haben oder NC-NH- oder
$R^3$ und $R^4$ zusammen eine C-N-Bindung bedeuten,

oder einem Säureadditionssalz einer solchen Verbindung

0001546

Europäisches Patentamt

**EUROPÄISCHER RECHERCHENBERICHT**

Nummer der Anmeldung

EP 78 10 0615

| Kategorie | EINSCHLÄGIGE DOKUMENTE<br>Kennzeichnung des Dokuments mit Angabe, soweit erforderlich, der maßgeblichen Teile | betrifft Anspruch | KLASSIFIKATION DER ANMELDUNG (Int.Cl.²) |
|---|---|---|---|
| X | US - A - 3 428 653 (STERLING DRUG)<br>* Spalte 1, Spalte 3, Zeilen 29-33 *<br>-- | 3,5 | C 07 D 209/14<br>A 61 K 31/40 |
| | US - A - 3 093 632 (CIBA)<br>* Spalte 4, Zeile 47 - Spalte 6, Zeile 22 *<br>-- | 3 | |
| | US - A - 3 317 560 (PHILIPS)<br>* Spalte 3, Zeile 25 - Spalte 4, Zeile 16 *<br>-- | 3 | RECHERCHIERTE SACHGEBIETE (Int. Cl.²)<br>A 61 K 31/40<br>C 07 D 209/14 |
| | US 3 354 174 (STERLING DRUG)<br>* Spalte 1, Zeilen 25-36; Spalte 5, Zeilen 35-45; Spalte 7, Zeilen 35-38; Spalten 19-20, Verbindung 256 *<br>-- | 1,3-5 | |
| | JOURNAL OF MEDICINAL CHEMISTRY, (1968) vol. 11, 844-848.<br>* Seite 844 *<br>---- | 4-5 | KATEGORIE DER GENANNTEN DOKUMENTE<br>X: von besonderer Bedeutung<br>A: technologischer Hintergrund<br>O: nichtschriftliche Offenbarung<br>P: Zwischenliteratur<br>T: der Erfindung zugrunde liegende Theorien oder Grundsätze<br>E: kollidierende Anmeldung<br>D: in der Anmeldung angeführtes Dokument<br>L: aus andern Gründen angeführtes Dokument |
| X | Der vorliegende Recherchenbericht wurde für alle Patentansprüche erstellt. | | &: Mitglied der gleichen Patentfamilie, übereinstimmendes Dokument |

| Recherchenort | Abschlußdatum der Recherche | Prüfer |
|---|---|---|
| Berlin | 20-11-1978 | FROELICH |

EPA form 1503.1 06.78